# EUROPÄISCHE PATENTSCHRIFT

(11) **EP 1 077 924 B1**
(45) Veröffentlichungstag und Bekanntmachung des Hinweises auf die Patenterteilung: **02.04.2003**
(21) Anmeldenummer: 99920841.6
(22) Anmeldetag: 04.05.1999
(51) Int. Cl.: C07C 219/06, C11D 1/62, C07C 217/50, A61K 7/50

(54) **ETHOXYLIERTE ESTERQUATS**
ETHOXYLATED QUATERNARY ESTER COMPOUNDS
COMPOSES QUATERNAIRES D'ESTER ETHOXYLES

(30) Priorität: 13.05.1998 DE 19821348
(43) Veröffentlichungstag der Anmeldung: 28.02.2001
(73) Patentinhaber: Cognis Deutschland GmbH & Co. KG, 40589 Düsseldorf (DE)
(72) Erfinder: BIGORRA LLOSAS, Joaquin, E-08203 Sabadell (ES); PI SUBIRANA, Rafael, E-08400 Granollers (ES); BONASTRE GILABERT, Nuria, E-08210 Barbera del Vallès (ES); WILSCH-IRRGANG, Anneliese, D-40593 Düsseldorf (DE)
(86) Internationale Anmeldenummer: EP9903000
(87) Internationale Veröffentlichungsnummer: WO99058492

(56) Entgegenhaltungen:
- EP-A- 0 267 551
- EP-A- 0 295 385
- EP-A- 0 739 976
- EP-A- 0 830 857
- DE-C- 4 308 794

## Beschreibung

### Gebiet der Erfindung

Die Erfindung befindet sich auf dem Gebiet der kationischen Tenside und deren Einsatz im Bereich der Kosmetik und Wäscheweichspülmittel. Gegenstand sind neue ethoxylierte Esterquats, die als gemeinsames Strukturmerkmal einen ethoxylierten Hydroxyacylrest aufweisen und sich in Wasser klar lösen.

### Stand der Technik

Unter der Bezeichnung "Esterquats" werden im allgemeinen quaternierte Fettsäuretriethanolaminestersalze verstanden. Es handelt sich dabei um bekannte Stoffe, die man nach den einschlägigen Methoden der präparativen organischen Chemie erhalten kann. In diesem Zusammenhang sei auf die Internationale Patentanmeldung **WO 91/01295** (Henkel) verwiesen, nach der man Triethanolamin in Gegenwart von unterphosphoriger Säure mit Fettsäuren partiell verestert, Luft durchleitet und anschließend mit Dimethylsulfat oder anderen geeigneten Alkylierungsmitteln quaterniert. Aus der Deutschen Patentschrift **DE-C1 4308794** (Henkel) ist überdies ein Verfahren zur Herstellung fester Esterquats bekannt, bei dem man die Quaternierung von Triethanolaminestern in Gegenwart von geeigneten Dispergatoren, vorzugsweise Fettalkoholen, durchführt. Übersichten zu diesem Thema sind beispielsweise von R.Puchta et al. in **Tens.Surf.Det., 30, 186 (1993),** M.Brock in **Tens.Surf.Det. 30, 394 (1993),** R.Lagerman et al. in **J.Am.Oil.Chem. Soc., 71, 97 (1994)** sowie I.Shapiro in Cosm.Toil. **109, 77 (1994)** erschienen.

Der besondere Vorteil von Esterquats gegenüber konventionellen kationischen Tensiden vom Typ der Tetraalkylammoniumsalze besteht in ihrem höheren Leistungsvermögen und insbesondere ihrer leichteren biologischen Abbaubarkeit. Problematisch ist hingegen, daß handelsübliche Esterquats üblicherweise herstellungsbedingt als alkoholische Konzentrate oder in Form von Schuppen in den Handel gelangen, die sich in Wasser nicht klar lösen. Wasserklare Zubereitungen, die ohne den Zusatz weiterer Hilfsstoffe sofort beispielsweise als Haarconditioner oder Wäscheweichspülmittel eingesetzt werden können, werden jedoch vom Markt gewünscht.

Demzufolge hat die komplexe Aufgabe der Erfindung darin bestanden, neue Esterquat-Typen zur Verfügung zu stellen, die nicht nur die ausgezeichneten anwendungstechnischen Eigenschaften anderer Esterquats aufweisen, sondern sich zusätzlich auch noch klar und spontan in kaltem Wasser lösen.

### Beschreibung der Erfindung

Ein erster Gegenstand der Erfindung betrifft Esterquats der Formel **(I),** 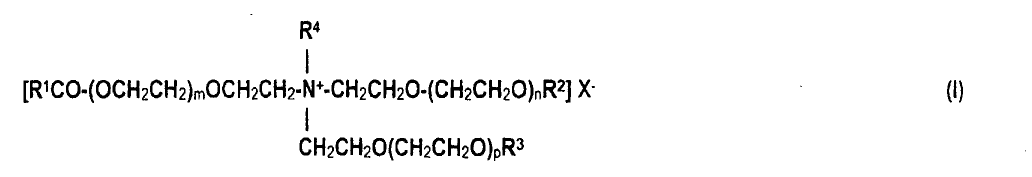 in der R¹CO für einen mit 1 bis 50 Mol Ethylenoxid ethoxylierten Hydroxyacylrest mit 16 bis 22 Kohlenstoffatomen, R² und R³ unabhängig voneinander für Wasserstoff oder R¹CO, R⁴ für einen Alkylrest mit 1 bis 4 Kohlenstoffatomen oder eine (CH₂CH₂O)_{q}H-Gruppe, m, n und p in Summe für 0 oder Zahlen von 1 bis 12, q für Zahlen von 1 bis 12 und X für Halogenid, Alkylsulfat oder Alkylphosphat steht.

Ein zweiter Gegenstand der Erfindung betrifft Esterquats der Formel **(II),** 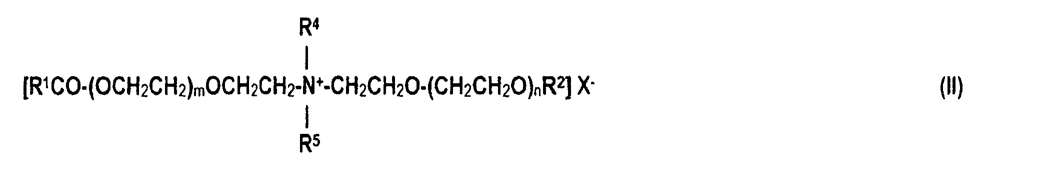 in der R¹CO für einen mit 1 bis 50 Mol Ethylenoxid ethoxylierten Hydroxyacylrest mit 16 bis 22 Kohlenstoffatomen, R² für Wasserstoff oder R¹CO, R⁴ und R⁵ unabhängig voneinander für Alkylreste mit 1 bis 4 Kohlenstoffatomen, m und n in Summe für 0 oder Zahlen von 1 bis 12 und X für Halogenid, Alkylsulfat oder Alkylphosphat steht.

Ein dritter Gegenstand der Erfindung betrifft Esterquats der Formel **(III),** 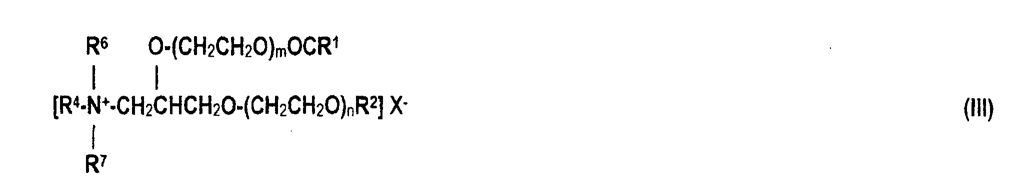 in der R¹CO für einen mit 1 bis 50 Mol Ethylenoxid ethoxylierten Hydroxyacylrest mit 16 bis 22 Kohlenstoffatomen, R² für Wasserstoff oder R¹CO, R⁴, R⁶ und R⁷ unabhängig voneinander für Alkylreste mit 1 bis 4 Kohlenstoffatomen, m und n in Summe für 0 oder Zahlen von 1 bis 12 und X für Halogenid, Alkylsulfat oder Alkylphosphat steht.

Die drei verschiedenen Esterquat-Typen **(I)** bis **(III)** eint der erfinderische Gedanken eines ethoxylierten Hydroxyacylrestes als zentralem Strukturelement. Überraschenderweise wurde gefunden, daß Esterquats der genannten Struktur nicht nur synthetischen wie natürlichen Fasern einen angenehmen Weichgriff verleihen, die statische Aufladung zwischen den Faserfibrillen signifikant vermindern, auf der Haut ein sensorisch leichtes Gefühl hinterlassen und zudem leicht biologisch abbaubar sind, sondern sich auch in kaltem Wasser spontan und ohne Trübung auflösen. Demzufolge erfüllen die neuen Esterquats das komplexe Anforderungsprofil in nahezu perfekter Weise. Typische Beispiele für Esterquats, auf die sich die Erfindung bezieht, sind Produkte auf Basis von Ricinolsäure, 12-Hydroxystearinsäure oder deren Mischungen im molaren Verhältnis 10 : 90 bis 90 : 10. Aus anwendungstechnischer Sicht haben sich Esterquats der Formeln **(I)** bis **(III)** als besonders vorteilhaft erwiesen, in der R¹CO für einen ethoxylierten Ricinolsäurerest, R² für R¹CO, R³ für Wasserstoff, R⁴ für eine Methylgruppe, m, n und p für 0 und X für Methylsulfat steht.

### Verfahren

Das Verfahren zur Herstellung umfaßt die Schritte der Ethoxylierung, Esterbildung und Alkylierung, wobei die Abfolge der Schritte austauschbar ist. Demzufolge betrifft ein weiterer Gegenstand der Erfindung ein Verfahren zur Herstellung von ethoxylierten Esterquats, bei dem man
(a) Hydroxycarbonsäureester mit 16 bis 22 Kohlenstoffatomen mit 1 bis 50 Mol Ethylenoxid ethoxyliert,
(b) die resultierenden ethoxylierten Hydroxycarbonsäureester mit Alkanolaminen oder Alkanolaminethoxylaten umestert und
(c) die resultierenden ethoxylierten Hydroxycarbonsäureesteramine mit Alkylierungsmitteln quaterniert.

Ein weiterer Gegenstand der Erfindung betrifft ein Verfahren zur Herstellung von ethoxylierten Esterquats, bei dem man
(a) Hydroxycarbonsäuren mit 16 bis 22 Kohlenstoffatomen mit Alkanolaminen oder Alkanolaminethoxylaten verestert,
(b) die resultierenden Hydroxycarbonsäureesteramine mit 1 bis 50 Mol Ethylenoxid ethoxyliert und
(c) die resultierenden ethoxylierten Hydroxycarbonsäureesteramine mit Alkylierungsmitteln quaterniert.

Ein weiterer Gegenstand der Erfindung betrifft schließlich ein Verfahren zur Herstellung von ethoxylierten Esterquats, bei dem man
(a) Hydroxycarbonsäuren mit 16 bis 22 Kohlenstoffatomen mit Alkanolaminen oder Alkanolaminethoxylaten verestert,
(b) die resultierenden Hydroxycarbonsäureesteramine mit Alkylierungsmitteln quaterniert und
(c) die resultierenden quaternierten Hydroxycarbonsäureesteramine mit 1 bis 50 Mol Ethylenoxid ethoxyliert.

Optimale Umsätze erhält man, wenn man der Reaktionssequenz "Ethoxylierung - Veresterung - Quaternierung" folgt, die daher auch bevorzugt ist. Als Einsatzstoffe sind hierbei Methylester und insbesondere Glycerinvoll- und/oder -partialester bevorzugt. So werden typischerweise ungehärtetes, teilweise oder vollständig gehärtetes Ricinusöl eingesetzt. Bei der Umesterung freigesetzter Alkohol, vorzugsweise Glycerin kann im Produkt als Lösemittel verbleiben.

### Carbonsäurekomponente

Die Carbonsäurekomponente der neuen Esterquats leitet sich vorzugsweise von der Ricinolsäure, der 12-Hydroxystearinsäure und/oder deren Gemischen im molaren Verhältnis 10 : 90 bis 90 ; 10 und insbesondere 25 : 75 bis 75 : 25 ab. In Abhängigkeit der gewählten Verfahrensvarianten können auch deren Anlagerungsprodukte mit 1 bis 50, vorzugsweise 3 bis 30 und insbesondere 10 bis 20 Mol Ethylenoxid eingesetzt werden, die nach den Verfahren des Stands der Technik erhältlich sind und eine konventionell breite oder eine eingeengte Homologenverteilung aufweisen können. Im Sinne des bevorzugten erfindungsgemäßen Verfahrens können anstelle der Ester grundsätzlich auch ebenfalls die freien Säuren oder Mischungen von Estern, speziell Triglyceriden, und Säuren eingesetzt werden. Dabei ist jedoch zu berücksichtigen, daß unter Einsatz konventioneller Katalysatoren die Ethoxylierung vorzugsweise an der primären und nicht an der sekundären Hydroxylgruppe stattfindet und die Anlagerung von Ethylenoxid daher leichter an der Säurefunktion als wie gewünscht an der Hydroxylgruppe der Fettkette erfolgt.

Es ist ferner möglich, die Hydroxycarbonsäuren bzw. deren Ethylenoxidanlagerungsprodukte in Abmischung mit weiteren Carboxylverbindungen einzusetzen, als da sind:
(a) aliphatische, lineare oder verzweigte, gesättigte oder ungesättigte Carbonsäuren mit 6 bis 22 Kohlenstoffatomen, wie z.B. Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Reduktion von Aldehyden aus der Roelen'schen Oxosynthese oder als Monomerfraktion bei der Dimerisierung von ungesättigten Fettsäuren anfallen. Bevorzugt sind technische, gesättigte oder teilgesättigte Fettsäuren mit 12 bis 18 Kohlenstoffatomen wie beispielsweise Kokos-, Palm-, Palmkem- oder Talgfettsäure.
(b) aliphatische Dicarbonsäuren mit 1 bis 12 Kohlenstoffatomen, wie z.B. Bernsteinsäure, Maleinsäure, Itaconsäure, 1,12- Dodecandisäure und insbesondere Adipinsäure;
(c) ein- oder mehrwertige Hydroxycarbonsäuren mit 2 bis 12 Kohlenstoffatomen, wie z.B. Milchsäure, Weinsäure, Äpfelsäure und insbesondere Citronensäure,
sowie deren Mischungen. Üblicherweise setzt man die Hydroxycarbonsäuren bzw. deren Ester und die Carboxylverbindungen - jeweils bezogen auf die Carboxylgruppen - im molaren Verhältnis 1 : 1 bis 10 : 1, vorzugsweise 2:1 bis 8 : 1 und insbesondere 3:1 1 bis 5:1 ein.

### Alkanolaminkomponente

Als Alkanolaminkomponenten können Triethanolamin (TEA), Methyldiethanolamin (MDA), oder deren Mischungen im Molverhältnis 5 : 95 bis 95 : 5 eingesetzt werden. Des weiteren kommen die Addukte von durchschnittlich 1 bis 10 Mol Ethylenoxid an die genannten Alkanolamine in Betracht. Die Hydroxycarbonsäuren bzw. deren Ester, gegebenenfalls im Gemisch mit den weiteren Carboxylverbindungen und die Alkanolamine können im molaren Verhältnis - bezogen auf Carboxylgruppen - von 1,1 : 1 bis 3 : 1 eingesetzt werden. Im Hinblick auf die anwendungstechnischen Eigenschaften der Esterquats hat sich ein Einsatzverhältnis von 1,2: 1 bis 2,2 : 1, vorzugsweise 1,5 : 1 bis 1,9 : 1 als besonders vorteilhaft erwiesen. Die bevorzugten Esterquats stellen technische Mischungen von Mono-, Di- und Triestern mit einem durchschnittlichen Veresterungsgrad von 1,5 bis 1.9 dar.

### Veresterung

Die Veresterung der Carbonsäure- mit den Alkanolaminkomponenten kann in an sich bekannter Weise durchgeführt werden. Als optimaler Katalysator für die Veresterung hat sich unterphosphorige Säure bzw. Natriumhypophosphit in Mengen von 0,01 bis 1 Gew.-% - bezogen auf die Einsatzstoffe - bewährt. Im Verlauf der Aufarbeitung, insbesondere bei höheren Temperaturen, kann es jedoch zu einer Zersetzung der Phosphorverbindungen und zur Bildung von kleinen Mengen an Phosphinen kommen, die ihrerseits zu einer geruchlichen Beeinträchtigung des Produktes Anlaß geben können.

Wie in der **EP-B1 0689533** (Henkel) beschrieben, können daher als Stabilisatoren Peroxidverbindungen eingesetzt werden. Zu diesem Zweck kommt neben Percarbonsäuren und Percarbonaten vorzugsweise Wasserstoffperoxid in Betracht. Unter Alkaliboranaten sind Lithium-, Kalium- und insbesondere Natriumboranat zu verstehen. Vorteilhafterweise werden die Peroxidverbindungen und die Alkaliboranate jeweils in Mengen von 0,005 bis 0,1, vorzugsweise 0,03 bis 0,06 Gew.-% - bezogen auf die Veresterungsprodukte - eingesetzt. Die weitere Stabilisatoren können Phenolderivate, wie z.B. Bisalkylhydroxytoluole und/oder Bisalkylanisole, insbesondere 2,6-Di-tert.Butyl-4-methyltoluol und 2,6-Di-tert. Butylanisol eingesetzt werden.

### Alkylierung und Alkylierungsmittel

Auch die Alkylierung der Alkanolaminester kann in an sich bekannter Weise durchgeführt werden, wie dies beispielsweise in der Deutschen Patentschrift **DE-C1 4409322** (Henkel) beschrieben wird. Die Alkanolaminester und die Quatemierungsmittel werden üblicherweise im molaren Verhältnis 1 : 0,95 bis 1 : 1,2, vorzugsweise 1 : 1 bis 1 : 1,1 eingesetzt. Als Quatemierungsmittel kommen Alkylhalogenide, wie beispielsweise Methylchlorid oder Dialkylsulfate, vorzugsweise Dimethylsulfate sowie Dialkylcarbonate, wie z.B. Dimethylcarbonat in Frage; die Quatemierungsprodukte haben dann eine kationische Natur. Es ist ferner möglich, die Quaternierung auch mit Halogencarbonsäuren, vorzugsweise Chloressigsäure oder deren Alkalisalzen durchzuführen, wobei dann amphotere Verbindungen entstehen. In der Regel wird die Alkylierung bzw. Quaternierung in einem inerten organischen Lösemittel, vorzugsweise einem niederen Alkohol, wie etwa Isopropylalkohol durchgeführt. Die resultierenden Esterquats kommen dann als alkoholische Konzentrate in den Handel oder werden nachträglich vom Lösemittel befreit. Es ist jedoch auch möglich, die Quaternierung lösungsmittelfrei oder in Wasser durchzuführen, wie dies beispielsweise in der **WO 95/20566** (Henkel) beschrieben wird. In einer besonderen Ausführungsform der Erfindung findet die Quatemierung jedoch in Gegenwart von Fettalkoholen bzw. Polyolen statt. Dies hat den Vorteil. daß in der Regel feste, schuppbare Produkte erhalten werden, bei denen das "Lösemittel" nicht abgetrennt werden muß, da es in der späteren Verwendung eine selbstständige Aufgabe, beispielsweise als Emulgator oder Dispergator übernimmt. Typische Beispiele für geeignete Fettalkohole sind Laurylalkohol, Myristylalkohol, Cetylalkohol, Stearylalkohol, Cetearylalkohol sowie deren technische Gemische, wie z.B. Kokosfettalkohol, Palmfettalkohol oder Talgfettalkohol. Als Polyole kommen beispielsweise Ethylenglycol, Propylenglycol, Glycerin, Partialglyceride, Alkylglucoside und dergleichen in Betracht. Ein entsprechendes Verfahren ist beispielsweise aus der Druckschrift **WO 94/21592** (Henkel) bekannt. Vorzugsweise wird die Quaternierung bei Temperaturen im Bereich von 70 bis 100 und insbesondere 80 bis 90°C durchgeführt. Die Reaktionszeit kann 1 bis 24 und vorzugsweise 2 bis 8 h betragen. Im Anschluß an die Quaternierung empfiehlt es sich, nicht umgesetztes Alkylierungsmittel durch Zugabe von Ammoniak, Glycin oder Monoethanolamin zu zerstören.

### Alkoxylierung

Wie oben erläutert kann die Ethoxylierung auch auf der Stufe der Ester oder Quaternierungsprodukte durchgeführt werden. In diesem Fall legt man die Ausgangsstoffe zusammen mit 0,5 bis 2,5 Gew.-% Katalysator (z.B. Natriummethylat oder calcinierter Hydrotalcit) in einen Rührautoklaven vor, evakuiert und gibt bei Temperaturen im Bereich von 120 bis 180 °C innerhalb von 1 bis 2 h die gewünschte Menge Ethylenoxid auf, wobei der autogene Druck bis auf 5 bar ansteigen kann. Anschließend läßt man noch etwa 30 min nachreagieren, kühlt den Reaktor ab, entspannt und neutralisiert den basischen Katalysator z.B. durch Zugabe von Milchsäure oder Phosphorsäure. Gegebenenfalls werden unlösliche Katalysatoren über eine Filterpresse abgetrennt.

### Gewerbliche Anwendbarkeit

Neben den geschilderten anwendungstechnischen Eigenschaften besteht der besondere Vorteil der neuen Esterquats darin, daß sie sich spontan und trübungsfrei auch in kaltem Wasser lösen. Weitere Gegenstände der Erfindung betreffen daher ihre Verwendung zur Herstellung von kosmetischen und/oder pharmazeutischen Zubereitungen sowie zur Herstellung von klaren Wäscheweichspül- und Flüssigwaschmitteln. Ein weiterer Vorteil besteht darin, daß die neuen Esterquats aniontensidverträglich sind, d.h. mit anionischen Tensiden nicht unter Fällung von Salzen abreagieren. Dies eröffnet die Möglichkeit zur Herstellung von Flüssigwaschmitteln mit Avivagewirkung.

### Kosmetische und/oder pharmazeutische Zubereitungen

Die erfindungsgemäßen Esterquats können zur Herstellung einer Vielzahl von Zubereitungen dienen, wie beispielsweise Haarshampoos, Haarlotionen, Schaumbäder, Cremes, Lotionen oder Salben. Ein weiterer Gegenstand der Erfindung betrifft demnach Zubereitungen der genannten Art, welche als weitere Hilfs- und Zusatzstoffe milde Co-Tenside, Ölkörper, Emulgatoren, Überfettungsmittel, Perlglanzwachse, Stabilisatoren, Konsistenzgeber, Verdickungsmittel, Polymere, Siliconverbindungen, biogene Wirkstoffe, Antischuppenmittel, Filmbildner, Konservierungsmittel, Hydrotrope, Solubilisatoren, UV-Lichtschutzfilter, Antioxidantien, Insektenrepellentien, Selbstbräuner, Parfümöle, Farbstoffe und dergleichen enthalten.

Typische Beispiele für geeignete milde, d.h. besonders hautverträgliche **Tenside** sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, Ethercarbonsäuren, Alkyloligoglucoside, Fettsäureglucamide, Alkylamidobetaine und/oder Proteinfettsäurekondensate, letztere vorzugsweise auf Basis von Weizenproteinen.

Als **Ölkörper** kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen C₆-C₂₂-Fettalkoholen, Ester von verzweigten C₆-C₁₃-Carbonsäuren mit linearen C₆-C₂₂-Fettalkoholen, Ester von linearen C₆-C₂₂-Fettsäuren mit verzweigten Alkoholen, insbesondere 2-Ethylhexanol, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, Guerbetcarbonate, Ester der Benzoesäure mit linearen und/oder verzweigten C₆-C₂₂-Alkoholen (z.B. Finsolv® TN), lineare oder verzweigte, symmetrische oder unsymmetrische Dialkylether mit 6 bis 22 Kohlenstoffatomen pro Alkylgruppe, Ringöffnungsprodukte von epoxidierten Fettsäureestern mit Polyolen, Silicon-öle und/oder aliphatische bzw. naphthenische Kohlenwasserstoffe in Betracht.

Als **Emulgatoren** kommen beispielsweise nichtionogene Tenside aus mindestens einer der folgenden Gruppen in Frage:
(1) Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
(2) C_{12/18}-Fettsäuremono- und -diester von Anlagerungsprodukten von 1 bis 30 Mol Ethylenoxid an Glycerin;
(3) Glycerinmono- und -diester und Sorbitanmono- und -diester von gesättigten und ungesättigten Fettsäuren mit 6 bis 22 Kohlenstoffatomen und deren Ethylenoxidanlagerungsprodukte;
(4) Alkylmono- und -oligoglycoside mit 8 bis 22 Kohlenstoffatomen im Alkylrest und deren ethoxylierte Analoga;
(5) Anlagerungsprodukte von 15 bis 60 Mol Ethylenoxid an Ricinusöl und/oder gehärtetes Ricinusöl;
(6) Polyol- und insbesondere Polyglycerinester, wie z.B. Polyglycerinpolyricinoleat, Polyglycerinpoly-12-hydroxystearat oder Polyglycerindimerat. Ebenfalls geeignet sind Gemische von Verbindungen aus mehreren dieser Substanzklassen;
(7) Anlagerungsprodukte von 2 bis 15 Mol Ethylenoxid an Ricinusöl und/oder gehärtetes Ricinusöl;
(8) Partialester auf Basis linearer, verzweigter, ungesättigter bzw. gesättigter C_{6/22}-Fettsäuren, Ricinolsäure sowie 12-Hydroxystearinsäure und Glycerin, Polyglycerin, Pentaerythrit, Dipentaerythrit, Zuckeralkohole (z.B. Sorbit), Alkylglucoside (z.B. Methylglucosid, Butylglucosid, Laurylglucosid) sowie Polyglucoside (z.B. Cellulose);
(9) Mono-, Di- und Trialkylphosphate sowie Mono-, Di- und/oder Tri-PEG-alkylphosphate und deren Salze;
(10) Wollwachsalkohole;
(11) Polysiloxan-Polyalkyl-Polyether-Copolymere bzw. entsprechende Derivate;
(12) Mischester aus Pentaerythrit, Fettsäuren, Citronensäure und Fettalkohol gemäß **DE-PS 1165574** und/oder Mischester von Fettsäuren mit 6 bis 22 Kohlenstoffatomen, Methylglucose und Polyolen, vorzugsweise Glycerin oder Polyglycerin sowie
(13) Polyalkylenglycole.

Die Anlagerungsprodukte von Ethylenoxid und/oder von Propylenoxid an Fettalkohole, Fettsäuren, Alkylphenole, Glycerinmono- und -diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind aus **DE-PS 2024051** als Rückfettungsmittel für kosmetische Zubereitungen bekannt.

C_{8/18}-Alkylmono- und -oligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 C-Atomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C_{12/18}-Acylsarcosin. Neben den ampholytischen kommen auch quartäre Emulgatoren in Betracht, wobei solche vom Typ der Esterquats, vorzugsweise methylquaternierte Difettsäuretriethanolaminester-Salze, besonders bevorzugt sind.

Als **Überfettungsmittel** können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

Als **Perlglanzwachse** kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxysubstituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensaure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

Als **Konsistenzgeber** kommen in erster Linie Fettalkohole oder Hydroxyfettalkohole mit 12 bis 22 und vorzugsweise 16 bis 18 Kohlenstoffatomen und daneben Partialglyceride. Fettsäuren oder Hydroxyfettsäuren in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete **Verdickungsmittel** sind beispielsweise Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono- und -diester von Fettsäuren, Polyacrylate, (z.B. Carbopole® von Goodrich oder Synthalene® von Sigma), Polyacrylamide, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

Geeignete **kationische Polymere** sind beispielsweise kationische Cellulosederivate, wie z.B. eine quaternierte Hydroxyethylcellulose, die unter der Bezeichnung Polymer JR 400® von Amerchol erhältlich ist, kationische Stärke, Copolymere von Diallylammoniumsalzen und Acrylamiden, quatemierte Vinylpyrrolidon/Vinylimidazol-Polymere, wie z.B. Luviquat® (BASF), Kondensationsprodukte von Polyglycolen und Aminen, quaternierte Kollagenpolypeptide, wie beispielsweise Lauryldimonium hydroxypropyl hydrolyzed collagen (Lamequat®L/Grünau), quatemierte Weizenpolypeptide, Polyethylenimin, kationische Siliconpolymere, wie z.B. Amidomethicone, Copolymere der Adipinsäure und Dimethylaminohydroxypropyldiethylentriamin (Cartaretine®/Sandoz), Copolymere der Acrylsäure mit Dimethyldiallylammoniumchlorid (Merquat® 550/Chemviron), Polyaminopolyamide, wie z.B. beschrieben in der **FR-A 2252840** sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar® CBS, Jaguar® C-17, Jaguar® C-16 der Firma Celanese, quaternierte Ammoniumsalz-Polymere, wie z.B. Mirapol® A-15, Mirapol® AD-1, Mirapol® AZ-1 der Firma Miranol.

Als **anionische, zwitterionische, amphotere und nichtionische Polymere** kommen beispielsweise Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Maleinsäureanhydrid-Copolymere und deren Ester, unvernetzte und mit Polyolen vemetzte Polyacrylsäuren, Acrylamidopropyltrimethylammoniumchlorid/Acrylat-Copolymere, Octylacrylamid/Methylmethacrylat/tert.Butylaminoethylmethacrylat/2-Hydroxypropylmethacrylat-Copolymere, Polyvinylpyrrolidon, Vinylpyrrolidon/Vinylacetat-Copolymere, Vinylpyrrolidon/Dimethylaminoethylmethacrylat/Vinylcaprolactam-Terpolymere sowie gegebenenfalls derivatisierte Celluloseether und Silicone in Frage.

Geeignete **Siliconverbindungen** sind beispielsweise Dimethylpolysiloxane, Methylphenylpolysiloxane, cyclische Silicone sowie amino-, fettsäure-, alkohol-, polyether-, epoxy-, fluor-, glykosid- und/oder alkylmodifizierte Siliconverbindungen. die bei Raumtemperatur sowohl flüssig als auch harzförmig vorliegen können. Typische Beispiele für **Fette** sind Glyceride, als **Wachse** kommen u.a. Bienenwachs, Carnaubawachs, Candelillawachs, Montanwachs, Paraffinwachs oder Mikrowachse gegebenenfalls in Kombination mit hydrophilen Wachsen, z.B. Cetylstearylalkohol oder Partialglyceriden in Frage. Als **Stabilisatoren** können Metallsalze von Fettsäuren, wie z.B. Magnesium-, Aluminium- und/oder Zinkstearat bzw. -ricinoleat eingesetzt werden. Unter **biogenen Wirkstoffen** sind beispielsweise Tocopherol, Tocopherolacetat, Tocopherolpalmitat, Ascorbinsäure, Desoxyribonucleinsäure, Retinol, Bisabolol, Allantoin, Phytantriol, Panthenol, AHA-Säuren, Aminosäuren, Ceramide, Pseudoceramide, essentielle Öle, Pflanzenextrakte und Vitaminkomplexe zu verstehen. Als **Antischuppenmittel** können Climbazol, Octopirox und Zinkpyrethion eingesetzt werden. Gebräuchliche **Filmbildner** sind beispielsweise Chitosan, mikrokristallines Chitosan, quaterniertes Chitosan, Polyvinylpyrrolidon, Vinylpyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen. Als **Quellmittel** für wäßrige Phasen können Montmorillonite, Clay Mineralstoffe, Pemulen sowie alkylmodifizierte Carbopoltypen (Goodrich) dienen.

Unter **UV-Lichtschutzfiltern** sind organische Substanzen zu verstehen, die in der Lage sind, ultraviolette Strahlen zu absorbieren und die aufgenommene Energie in Form längerwelliger Strahlung, z.B. Wärme wieder abzugeben. UVB-Filter können öllöslich oder wasserlöslich sein. Als öllösliche Substanzen sind z.B. zu nennen:
- 3-Benzylidencampher bzw. 3-Benzylidennorcampher und dessen Derivate, z.B. 3-(4-Methylbenzyliden)campher wie in der **EP-B1 0693471** beschrieben;
- 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)benzoesäureamylester;
- Ester der Zimtsäure, vorzugsweise 4-Methoxyzimtsäure-2-ethylhexylester, 4-Methoxyzimtsäurepropylester, 4-Methoxyzimtsäureisoamylester 2-Cyano-3-phenyl-zimtsäure-2-ethylhexylester (Octocrylene);
- Ester der Salicylsäure, vorzugsweise Salicylsäure-2-ethylhexylester, Salicylsäure-4-isopropylbenzylester, Salicylsäurehomomenthylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzmalonsäuredi-2-ethylhexylester;
- Triazinderivate, wie z.B. 2,4,6-Trianilino-(p-carbo-2'-ethyl-1'-hexyloxy)-1,3,5-triazin und Octyltriazon, wie in der **EP-A10818450** beschrieben;
- Propan-1,3-dione, wie z.B. 1-(4-tert.Butylphenyl)-3-(4methoxyphenyl)propan-13-dion;
- Ketotricyclo(5.2.1.0)decan-Derivate, wie in der **EP-B1 0694521** beschrieben.

Als wasserlösliche Substanzen kommen in Frage:
- 2-Phenylbenzimidazol-5-sulfonsäure und deren Alkali-, Erdalkali-, Ammonium-, Alkylammonium-, Alkanolammonium- und Glucammoniumsalze;
- Sulfonsäurederivate von Benzophenonen, vorzugsweise 2-Hydroxy-4-methoxybenzophenon-5-sulfonsäure und ihre Salze;
- Sulfonsäurederivate des 3-Benzylidencamphers, wie z.B. 4-(2-Oxo-3-bornylidenmethyl)benzolsulfonsäure und 2-Methyl-5-(2-oxo-3-bomyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan (Parsol 1789), oder 1-Phenyl-3-(4'-isopropylphenyl)-propan-1,3-dion. Die UV-A und UV-B-Filter können selbstverständlich auch in Mischungen eingesetzt werden. Neben den genannten löslichen Stoffen kommen für diesen Zweck auch unlösliche Pigmente, nämlich feindisperse Metalloxide bzw. Salze in Frage, wie beispielsweise Titandioxid, Zinkoxid, Eisenoxid, Aluminiumoxid, Ceroxid, Zirkoniumoxid, Silicate (Talk), Bariumsulfat und Zinkstearat. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Weitere geeignete UV-Lichtschutzfilter sind der Übersicht von P.Finkel in **SÖFW-Journal 122, 543 (1996)** zu entnehmen.

Neben den beiden vorgenannten Gruppen primärer Lichtschutzstoffe können auch sekundäre Lichtschutzmittel vom Typ der **Antioxidantien** eingesetzt werden, die die photochemische Reaktionskette unterbrechen, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester ) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z B. γ-Linolensäure, Linolsäure. Ölsäure), Folsäure und deren Derivate, Ubichinon und Ubichinol und deren Derivate, Vitamin C und Derivate (z.B. Ascorbylpaimitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate (z.B. ZnO, ZnSO₄) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Zur Verbesserung des Fließverhaltens können ferner **Hydrotrope,** wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Typische Beispiele sind
- Glycerin;
- Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche mit 1 bis 8 Kohlenstoffen im Alkylrest, wie beispielsweise Methyl- und Butylglucosid;
- Zuckeralkohole mit 5 bis 12 Kohlenstoffatomen, wie beispielsweise Sorbit oder Mannit,
- Zucker mit 5 bis 12 Kohlenstoffatomen, wie beispielsweise Glucose oder Saccharose;
- Aminozucker, wie beispielsweise Glucamin.

Als **Konservierungsmittel** eignen sich beispielsweise Phenoxyethanol, Formaldehydlösung, Parabene, Pentandiol oder Sorbinsäure sowie die in Anlage 6, Teil A und B der Kosmetikverordnung aufgeführten weiteren Stoffklassen Als **Insekten-Repellentien** kommen N,N-Diethyl-m-touluamid, 1,2-Pentandiol oder Insect repellent 3535 in Frage, als **Selbstbräuner** eignet sich Dihydroxyaceton.

Als **Parfümöle** seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris. Calmus), Hölzern (Pinien-, Sandel-, Guajak-. Zedern-, Rosenholz), Kräutern und Gräsern (Estragon. Lemongras, Salbei. Thymian). Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethyl-phenylglycinat, Allylcyclohexylpropionat, Styrallylpro-pionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, α-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evemyl, Iraldein gamma, Phenylessigsäure, Geranylacetat, Benzylacetat, Rosenoxid, Romilllat, Irotyl und Floramat allein oder in Mischungen, eingesetzt.

Als **Farbstoffe** können die für kosmetische Zwecke geeigneten und zugelassenen Substanzen verwendet werden, wie sie beispielsweise in der Publikation **"Kosmetische Färbemittel" der Farbstoffkommission der Deutschen Forschungsgemeinschaft, Verlag Chemie, Weinheim, 1984, S.81-106** zusammengestellt sind. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-% - bezogen auf die Mittel - betragen. Die Herstellung der Mittel kann durch übliche Kalt- oder Heißprozesse erfolgen; vorzugsweise arbeitet man nach der Phaseninversionstemperatur-Methode.

### Klare Avivagemittel

Ein weiterer Gegenstand der Erfindung betrifft klare Avivagemittel mit einem Gehalt an den erfindungsgemäßen Esterquats und nichtionischen Tensiden. wobei die beiden Komponenten - bezogen auf Aktivsubstanz - im Gewichtsverhältnis 10 : 90 bis 90 : 10, vorzugsweise 25 : 75 bis 75 : 25 und insbesondere 40 : 60 bis 60 : 40 anwesend sein können. Der Begriff Avivagemittel bezieht sich dabei nicht nur auf Wäscheweichspülmittel, sondern auch auf industrielle Produkte zur Ausrüstung und Vorbehandlung von Gamen, Geweben, Gewirken, Stoffen und dergleichen. Typische Beispiele für geeignete nichtionische Tenside sind Fettalkoholpolyglycolether, Alkylphenolpolyglycolether, Fettsäurepolyglycolester, Fettsäureamidpolyglycolether, Fettaminpolyglycolether, alkoxylierte Triglyceride, Mischether bzw. Mischformale, gegebenenfalls partiell oxidierte Alk(en)yloligoglykoside bzw. Glucoron-säurederivate, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen. Aus anwendungstechnischer Sicht haben sich Avivagemittel besonders bewährt, die neben den Esterquats als nichtionische Tensidkomponente entweder Anlagerungsprodukte von durchschnittlich 1 bis 40 Mol Ethylenoxid an Fettalkohole bzw. Oxoalkohole mit 10 bis 18 Kohlenstoffatomen und/oder Alkyloligoglucoside enthalten. Hinsichtlich Struktur und Herstellung dieser Stoffe sei auf einschlägige Übersichtsarbeiten beispielsweise **J.Falbe (ed**.**), "Surfactants in Consumer Products", Springer Verlag, Berlin, 1987, S, 54-124** oder **J.Falbe (ed.), "Katalysatoren, Tenside und Mineralöladditive", Thieme Verlag, Stuttgart, 1978, S. 123-217** verwiesen.

Als weitere Einsatzstoffe können die Avivagemittel Hydrotrope enthalten, wie beispielsweise Ethanol, Isopropylalkohol, Ethylenglycol, Propylenglycol, Hexylenglycol, Glycerin und dergleichen. Die Avivagemittel können einen Aktivsubstanzanteil, d.h. nicht wäßrigen Anteil im Bereich von 5 bis 50 und vorzugsweise 15 bis 30 Gew.-% aufweisen.

### Flüssigwaschmittel mit Avivagewirkung

Flüssigwaschmittel, die die erfindungsgemäßen Esterquats enthalten, können neben den bereits genannten nichtionischen Tensiden insbesondere auch anionische Tenside sowie weitere typische Inhaltsstoffe, wie beispielsweise Builder, Enzyme, Enzymstabilisatoren, Bleichmittel, optische Aufheller, Verdickungsmittel, Soil repellants, Schauminhibitoren, Lösungsvermittler, anorganische Salze sowie Duft- und Farbstoffe aufweisen.

Typische Beispiele für **anionische Tenside** sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Fettsäureethersulfate, Hydroxymischethersulfate, Monoglycerid(ether)sulfate, Fettsäureamid(ether)-sulfate, Mono- und. Dialkyl-sulfosuccinate, Mono- und Dialkylsulfosuccinamate, Sulfotriglyceride. Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Die Flüssigwaschmittel können die Esterquats und die anionischen und/oder nichtionischen Tenside - bezogen auf Aktivsubstanz - im Gewichtsverhältnis 10 : 90 bis 90 : 10, vorzugsweise 25 : 75 bis 75 : 25 und insbesondere 40 : 60 bis 60 : 40 enthalten.

Geeignete **Builder** sind Zeolithe, Schichtsilicate, Phosphate sowie Ethylendiamintetraessigsäure, Nitrilotriessigsäure, Citronensäure sowie anorganische Phosphonsäuren. Als **Verdickungsmittel** können beispielsweise gehärtetes Rizinusöl, Salze von langkettigen Fettsäuren, die vorzugsweise in Mengen von 0 bis 5 Gew.-% und insbesondere in Mengen von 0,5 bis 2 Gew.-%, beispielsweise Natrium-, Kalium-, Aluminium-, Magnesium- und Titanstearate oder die Natrium- und/oder Kaliumsalze der Behensäure, sowie weitere polymere Verbindungen eingesetzt werden. Zu den letzten gehören bevorzugt Polyvinylpyrrolidon, Urethane und die Salze polymerer Polycarboxylate, beispielsweise homopolymerer oder copolymerer Polyacrylate, Polymethacrylate und insbesondere Copolymere der Acrylsäure mit Maleinsäure, vorzugsweise solche aus 50 bis 10% Maleinsäure. Die relative Molekülmasse der Homopolymeren liegt im allgemeinen zwischen 1000 und 100000, die der Copolymeren zwischen 2000 und 200000, vorzugsweise zwischen 50000 bis 120000, bezogen auf die freie Säure. Insbesondere sind auch wasserlösliche Polyacrylate geeignet, die beispielsweise mit etwa 1% eines Polyallylethers der Sucrose quervernetzt sind und die eine relative Molekülmasse oberhalb 1000000 besitzen. Beispiele hierfür sind unter dem Namen Carbopol® 940 und 941 erhältliche Polymere. Die quervernetzten Polyacrylate werden vorzugsweise in Mengen nicht über 1 Gew.-%, besonders bevorzugt in Mengen von 0,2 bis 0,7 Gew.-% eingesetzt.

Als **Enzyme** kommen - sofern in wäßrig/alkoholischer Umgebung beständig - solche aus der Klasse der Proteasen, Lipase, Amylasen, Cellulasen bzw. deren Gemische in Frage. Besonders gut geeignet sind aus Bakterienstämmen oder Pilzen, wie Bacillus subtilis, Bacillus lichenformis und Streptomyces griseus gewonnene enzymatische Wirkstoffe. Vorzugsweise werden Proteasen vom Subtilisin-Typ und insbesondere Proteasen, die aus Bacillus lentes gewonnen werden, eingesetzt. Ihr Anteil kann etwa 0,2 bis 2 Gew.-% betragen. Die Enzyme können an Trägerstoffen adsorbiert oder in Hüllsubstanzen eingebettet sein, um sie gegen vorzeitige Zersetzung zu schützen. Zusätzlich zu mono- und polyfunktionellen Alkoholen und Phosphonaten können die Mittel weitere **Enzymstabilisatoren** enthalten. Beispielsweise können 0,5 bis 1 Gew.-% Natriumformiat eingesetzt werden. Möglich ist auch der Einsatz von Proteasen, die mit löslichen Calciumsalzen und einem Calciumgehalt von vorzugsweise etwa 1,2 Gew.-%, bezogen auf das Enzym, stabilisiert sind. Besonders vorteilhaft ist jedoch der Einsatz von Borverbindungen, beispielsweise von Borsäure. Boroxid. Borax und anderen Alkalimetallboraten. wie den Salzen der Orthoborsäure (H₃BO₃), der Metaborsäure (HBO₂) und der Pyroborsäure (Tetraborsäure H₂B₄O₇). Beim Einsatz im maschinellen Waschverfahren kann es von Vorteil sein, den Mitteln übliche **Schauminhibitoren** zuzusetzen. Geeignete Schauminhibitoren enthalten beispielsweise bekannte Organopolysiloxane, Paraffine oder Wachse. Auch die Flüssigwaschmittel können einen Aktivsubstanzanteil, d.h. nicht wäßrigen Anteil im Bereich von 5 bis 50 und vorzugsweise 15 bis 30 Gew.-% aufweisen.

### Beispiele

**Beispiel 1.** In einem 2-I-Dreihalskolben mit Rührer, Destillationsaufsatz und Gaseinleitungsrohr wurden 845 g (0,49 Mol) eines Adduktes von durchschnittlich 18 Mol Ethylenoxid an Ricinusöl, 117 g (0,79 Mol) Triethanolamin, 17 g Ölsäure, 0,5 g Natriumborhydrid und 0,5 g Natriumhypophosphit vorgelegt. Die Reaktionsmischung wurde mit Stickstoff abgedeckt, auf 200°C erhitzt und bei dieser Temperatur über einen Zeitraum von 4 h gehalten, bis kein weiteres Kondensationswasser mehr abgeschieden wurde und die Säurezahl unter 1 abgesunken war. Anschließend wurden 900 g (0,74 Mol) des gebildeten Esters in eine 2-I-Rührappartur mit Tropftrichter und Intensivkühler überführt und dort bei 40°C portionsweise mit 88 g (0,7 Mol) Dimethylsulfat versetzt. Anschließend wurde die Mischung 4 h bei 65°C gerührt. Es wurde eine leicht gelb gefärbte transparente Flüssigkeit erhalten, die bei 20°C als 35 Gew.-%ige Zubereitung in Wasser klar löslich war.

**Beispiel 2.** Analog Beispiel 1 wurden 450 g (0,48 Mol) Ricinusöl, 450 g (0,26 Mol) Ricinolsäure+18EO, 206 g (1,38 Mol) Triethanolamin, 20 g Ölsäure, 0,9 g Natriumborhydrid und 0,5 g Natriumhypophosphit zur Reaktion gebracht. 1000 g (1,23 Mol) des resultierenden Esters wurde in 129 g Isopropylalkohol gelöst und mit 147 g (1,16 Mol) Dimethylsulfat quaterniert. Es wurde eine transparente Flüssigkeit mit einem Aktivsubstanzgehalt von 90 Gew.-% erhalten.

**Beispiel 3.** Analog Beispiel 2 wurden 450 g (0,48 Mol) gehärtetes Ricinusöl, 450 g (0,26 Mol) 12-Hydroxystearinsäure+18EO, 206 g (1,38 Mol) Triethanolamin, 20 g Ölsäure, 0,9 g Natriumborhydrid und 0,5 g Natriumhypophosphit zur Reaktion gebracht. 1000 g (1,23 Mol) des resultierenden Esters wurde in 129 g Isopropylalkohol gelöst und mit 147 g (1,16 Mol) Dimethylsulfat quaterniert. Nach Abtrennung des Lösemittels wurden weiße Schuppen mit einem Aktivsubstanzgehalt von 90 Gew.-% erhalten.

**Beispiel 4.** Bei 20°C wurde eine Mischung aus 300 g des Esterquats gemäß Beispiel 1, 80 g Ricinolsäure+36 EO und 20 g Kokosalkylglucosid hergestellt und diese mit Wasser auf 1000 ml verdünnt. Es wurde eine klare Lösung erhalten, die ausgezeichnete Avivageleistungen zeigte.

Weitere Formulierungsbeispiele für kosmetische Anwendungen mit den neuen Esterquats finden sich in der nachfolgenden Tabelle; alle Mengenangaben verstehen sich als Gew.-%.

| **Haarspülung (I)** | |
|---|---|
| Hexadecyl Polyglucose (and) Hexadecyl Alcohol | 4,0 |
| Hydrolyzed Keratin | 2,3 |
| Coco Glucosides | 2,0 |
| Esterquat gemäß Bsp. 1 | 1,0 |
| Polyglyceryl-3-isostearat | 1,0 |
| Decyl Oleate | 1,0 |
| Glyceryl Stearate | 0,5 |

| **Haarspülung (II)** | |
|---|---|
| Cetearyl Alcohol | 2,5 |
| Esterquat gemäß Bsp. 1 | 1,0 |
| Dicaprylyl Ether | 1,0 |
| Ceteareth-20 | 0,8 |
| Glyceryl Stearate | 0,5 |

| **Haarspülung (III)** | |
|---|---|
| Cetearyl Alcohol | 2,5 |
| Esterquat gemäß Bsp. 1 | 1,0 |
| Octyldodecanol | 1,0 |
| Ceteareth-20 | 0,8 |
| Glyceryl Stearate | 0,5 |

| **Haarspülung (IV)** | |
|---|---|
| Cetearyl Alcohol | 2,5 |
| Hydrolyzed Collagen | 2,0 |
| Esterquat gemäß Bsp. 1 | 1,0 |
| Polyglyceryl-3 Diisostearat | 1,0 |
| Decyl Oleate | 1,0 |
| Ceteareth-20 | 0,8 |
| Glyceryl Stearate | 0,5 |

| **Leave-on Hair Rinse** | |
|---|---|
| Polyacrylate (and) Laureth-2 (and) Paraffinöl | 3,0 |
| Hydrolyzed Collagen | 2,0 |
| Esterquat gemäß Bsp. 1 | 0,8 |
| Coco Glucosides | 0,5 |
| Oleyl Erucate | 0,5 |
| Tocophecol Acetate | 0,2 |
| Ethanol | 10,0 |
| Glycerin (86 %ig) | 5,0 |

| **Haarkur (I)** | |
|---|---|
| Cetearyl Alcohol | 3,0 |
| Soja Sterol | 1,0 |
| Esterquat gemäß Bsp. 1 | 1,0 |
| Ceteareth-20 | 0,8 |
| Glyceryl Stearate | 0,5 |

| **Haarkur (II)** | |
|---|---|
| Cetearyl Alcohol | 2,5 |
| Esterquat gemäß Bsp. 1 | 1,5 |
| Ceteareth-20 | 1,0 |
| Soja Sterol | 1,0 |
| Octyldodecanol | 1,0 |
| Glyceryl Sterate | 1,0 |

| **Duschbad (I)** | |
|---|---|
| Sodium Laureth Sulfate | 38,0 |
| Coco Glucosides | 7,0 |
| Glyceryl Laurate (and) Potassium Cocoyl Hydrolyzed Collagen | 3,0 |
| Laureth-2 | 3,0 |
| Esterquat gemäß Bsp. 1 | 0,5 |

| **Duschbad (II)** | |
|---|---|
| Sodium Laureth Sulfate | 38,0 |
| Coco Glucosides | 7,0 |
| Glycol Distearate (and) Laureth-4 (and) Cocoamidopropyl Betaine | 3,0 |
| Laureth-2 (NRE) | 3,0 |
| Glyceryl Laurate (and) Potassium Cocoyl Hydrolyzed Collagen | 2,0 |
| Esterquat gemäß Bsp. 1 | 0,5 |
| NaCI | 1,5 |

| **Duschgel** | |
|---|---|
| Sodium Laureth Sulfate | 25,0 |
| Disodium Laureth Sulfosuccinate | 10,0 |
| Cocamidopropyl Betaine | 10,0 |
| Coco Glucosides | 6,0 |
| Glycol Distearate (and) Laureth-4 (and) Cocamidopropyl Betaine | 5,0 |
| Glyceryl Laurate (and) Potassium Cocoyl Hydrolyzed Collagen | 4,0 |
| Propylene Glycol (and) PEG-55 Propylene Glycol Oleate | 1,5 |
| PEG-7 Glyceryl Cocoate | 1,0 |
| Laureth-2 | 1,0 |
| Esterquat gemäß Bsp. 1 | 0,5 |

| **Waschlotion** | |
|---|---|
| Coco Glucosides (and) Sodium Laureth Sulfate | 16,0 |
| Glycol Distearate (and) Laureth-4 (and) Cocamidopropyl Betaine | 5.0 |
| Esterquat gemäß Bsp. 1 | 0.5 |
| NaCl | 1.5 |

| **Duschbad "Two-in-one" (I)** | |
|---|---|
| Sodium Laureth Sulfate | 20,0 |
| Cocamidopropyl Betaine | 20,0 |
| Coco Glucosides | 5,0 |
| Hydrolyzed Collagen | 1,0 |
| Esterquat gemäß Bsp. 1 | 1,0 |
| Glycol Distearate (and) Laureth-4 (and) Cocamidopropyl Betaine | 5,0 |
| Sodium Styrene/Acrylates Copolymer | 2,0 |
| Laureth-2 (NRE) | 0,6 |

| **Duschbad "Two-in-one" (II)** | |
|---|---|
| Sodium Laureth Sulfate | 20,0 |
| Cocamidopropyl Betaine | 20,0 |
| Coco Glucosides | 5,0 |
| Esterquat gemäß Bsp. 1 | 1,5 |
| Glycol Distearate (and) Laureth-4 (and) Cocamidopropyl Betaine | 3,0 |
| PEG-7 Glyceryl Cocoate | 0,2 |
| Sodium Styrene/Acrylates Copolymer | 1,0 |
| Laureth-2 (NRE) | 0,6 |
| Glycerin (86 Gew.-%ig) | 5,0 |

| **Duschbad "Two-in-one" (III)** | |
|---|---|
| Sodium Laureth Sulfate | 12,4 |
| Lauryl Glucosides | 4,0 |
| Esterquat gemäß Bsp. 1 | 4,0 |
| Glycol Distearate (and) Laureth-4 (and) Cocamidopropyl Betaine | 4,0 |
| Panthenol | 1,0 |

| **Duschbad und Emulsion "Two-in-one"** | |
|---|---|
| Coco Glucosides (and) sodium Laureth Sulfate | 40,0 |
| Ceteareth-20 | 1,0 |
| Octyldodecanol | 3,0 |
| Polyglyceryl-2-PEG-4 Copolymer | 4,0 |
| Sodium Styrene/Acrylate Copolymer | 1,0 |
| Esterquat gemäß Bsp 1 | 1,0 |
| Parfümöl | 0,5 |

| **Shampoo (I)** | |
|---|---|
| Sodium Laureth Sulfate | 25,0 |
| Coco Glucosides | 5,0 |
| Cocamidopropyl Betaine | 8,0 |
| Esterquat gemäß Bsp. 1 | 3,0 |
| Laureth-2 (NRE) | 1,5 |
| PPG-2-Ceteareth-9 | 1,0 |
| Parfümöl | 5,0 |

| **Shampoo (II)** | |
|---|---|
| Sodium Laureth Sulfate | 11,0 |
| Disodium Laureth Sulfosuccinate | 7,0 |
| Coco Glucosides | 4,0 |
| Esterquat gemäß Bsp. 1 | 1,0 |
| Hydrolyzed Collagen | 2,0 |
| NaCl | 1,6 |

| **Shampoo (III)** | |
|---|---|
| Coco Glucosides (and) Sodium Laureth Sulfate | 16,0 |
| Esterquat gemäß Bsp. 1 | 2,0 |
| NaCI | 2,0 |

| **Shampoo (IV)** | |
|---|---|
| Coco Glucosides (and) Sodium Laureth Sulfate | 17,0 |
| Hydrolyzed Collagen | 2,0 |
| Esterquat gemäß Bsp. 1 | 2,0 |
| Glycerin (86 Gew.-%ig) | 1,0 |
| Triethyleneglycol Distearate (and) Sodium Laureth Sulfate | 3,0 |
| NaCl | 2,2 |

| **Shampoo (V)** | |
|---|---|
| Sodium Laureth Sulfate | 11,0 |
| Coco Glucosides | 6,0 |
| Hydrolyzed Collagen | 2,0 |
| Esterquat gemäß Bsp. 1 | 2,0 |
| Triethyleneglycol Distearate (and) Sodium Laureth Sulfate | 3,0 |
| NaCl | 3,0 |

| **Shampoo (VI)** | |
|---|---|
| Ammonium Laureth Sulfate | 23,0 |
| Coco Glucosides | 4,0 |
| Cocamidopropyl Betaine | 7,0 |
| Esterquat gemäß Bsp. 1 | 2,0 |
| Hydrogenated Tallow Glycerides (and) Potassium Cocoyl Hydrolyzed Collagen | 5,0 |
| Glyceryl Laurate | 1,,0 |
| NaCI | 3,0 |

| **Schaumbad (I)** | |
|---|---|
| Coco Glucosides (and) Sodium Laureth Sulfate | 22,0 |
| Cocamidopropyl Betaine | 15,0 |
| Esterquat gemäß Bsp. 1 | 3,0 |
| PEG-7 Glyceryl Cocoate | 2,0 |
| Glycol Distearate (and) Laureth-4 (and) Cocamidopropyl Betaine | 5,0 |

| **Schaumbad (II)** | |
|---|---|
| Sodium Laureth Sulfate | 30,0 |
| Cocamidopropyl Betaine | 10,0 |
| Coco Glucosides | 10,0 |
| Glyceryl Laurate (and) Potassium Cocoyl Hydrolyzed Collagen | 4,0 |
| Esterquat gemäß Bsp. 1 | 2,0 |
| Hydrolyzed Wheat Protein | 0,5 |

| **Schaumbad (III)** | |
|---|---|
| Melissenöl | 5,0 |
| PPG-7-Ceteareth-9 | 15,0 |
| Coco Glucosides | 30,0 |
| Cocamidopropyl Betaine | 10,0 |
| Esterquat gemäß Bsp. 1 | 4,0 |
| Propylene Glycol (and) PEG-55 Propylene Glycol Oleate | 3,8 |
| Laureth-2 (NRE) | 1,5 |

| **Schaumbad (IV)** | |
|---|---|
| Coco Glucosides (and) Sodium Laureth Sulfate | 22,0 |
| Cocamidopropyl Betaine | 15,0 |
| Esterquat gemäß Bsp. 1 | 2,0 |
| PEG-7 Glyceryl Cocoate | 2,0 |
| Glycol Distearate (and) Laureth-4 (and) Cocamidopropyl Betaine | 5,0 |

| **Schaumbadkonzentrat** | |
|---|---|
| Sodium Laureth Sulfate | 25,0 |
| Coco Glucosides | 20,0 |
| Cocamidopropyl Betaine | 20,0 |
| Esterquat gemäß Bsp. 1 | 5,0 |
| PEG-7 Glyceryl Cocoate | 5,0 |
| Hydrolyzed Collagen | 2,0 |
| PEG-60 Hydrogenated Castor Oil | 5,0 |
| Citronensäuren (50 gew -%ig) | 0,5 |

| **Softcreme** | |
|---|---|
| Glyceryl Stearate (and) Ceteareth-12/20 (and) Cetearyl Alcohol (and) Cetyl | 5,0 |
| Decyl Oleate | 3,0 |
| Cetearyl Isononanoate | 3,0 |
| Glycerin (86 Gew.-%ig) | 3,0 |
| Esterquat gemäß Bsp. 1 | 40,0 |

| **Feuchtigkeitsemulsion** | |
|---|---|
| Glyceryl Stearate (and) Ceteareth-12/20 (and) Cetearyl alcohol (and) Cetyl Palmitate | 5.0 |
| Decyl Oleate | 3,0 |
| Cetearyl Isononanoate | 3.0 |
| Glycerin (86 Gew.-%ig) | 3.0 |
| Esterquat gemäß Bsp. 1 | 60.0 |

| **Nachtcreme** | |
|---|---|
| Polyglyceryl-3 Diisostearate | 4,0 |
| Glyceryl Oleate | 2,0 |
| Bienenwachs | 7,0 |
| Dicaprylyl Ether | 5,0 |
| Octyldodecanol | 10,0 |
| Coco Caprylate Caprate | 5,0 |
| Glycerin (86 Gew.-%ig) | 5,0 |
| Magnesiumsulfat | 1,0 |
| Esterquat gemäß Bsp. 1 | 5,0 |

| **Sonnenschutzcreme** | |
|---|---|
| Cetearylglucoside (and) Cetearyl Alkohol | 3,0 |
| Esterquats gemäß Bsp. 1 | 1,0 |
| Hydrogenated Palm Glycerides | 2,0 |
| Di-n-octylcarbonat | 8,0 |
| Coco Glycerides | 6,0 |
| Octyl Methoxycinnamate | 5,0 |
| 4-Methylbenzyliden Camphor | 3,0 |
| Benzophenon-3 | 4,0 |
| Titandioxid | 1,0 |
| Zinkoxid | 1,0 |
| Octyl Triazone | 1,0 |
| Glycerin (86 Gew.-%ig) | 5,0 |

## Patentansprüche

1. Esterquats der Formel **(I),** 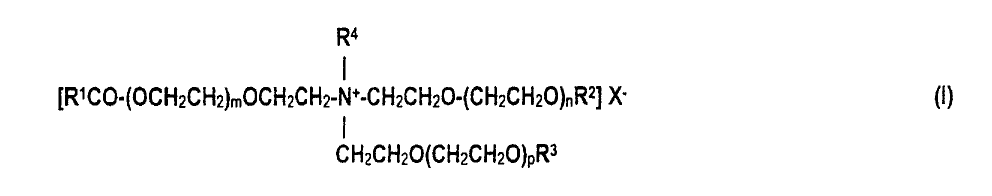 in der R¹CO für einen mit 1 bis 50 Mol Ethylenoxid ethoxylierten Hydroxyacylrest mit 16 bis 22 Kohlenstoffatomen, R² und R³ unabhängig voneinander für Wasserstoff oder R¹CO, R⁴ für einen Alkylrest mit 1 bis 4 Kohlenstoffatomen oder eine (CH₂CH₂O)_{q}H-Gruppe, m, n und p in Summe für 0 oder Zahlen von 1 bis 12, q für Zahlen von 1 bis 12 und X für Halogenid, Alkylsulfat oder Alkylphosphat steht.

2. Esterquats der Formel **(II),** 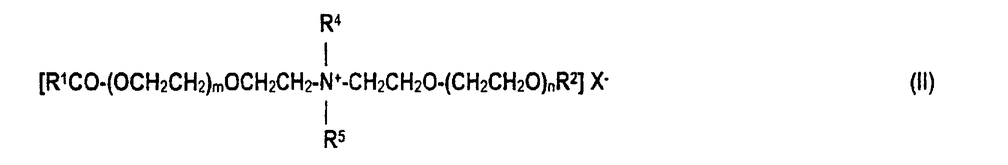 in der R¹CO für einen mit 1 bis 50 Mol Ethylenoxid ethoxylierten Hydroxyacylrest mit 16 bis 22 Kohlenstoffatomen, R² für Wasserstoff oder R¹CO, R⁴ und R⁵ unabhängig voneinander für Alkylreste mit 1 bis 4 Kohlenstoffatomen, m und n in Summe für 0 oder Zahlen von 1 bis 12 und X für Halogenid, Alkylsulfat oder Alkylphosphat steht.

3. Esterquats der Formel **(III),** 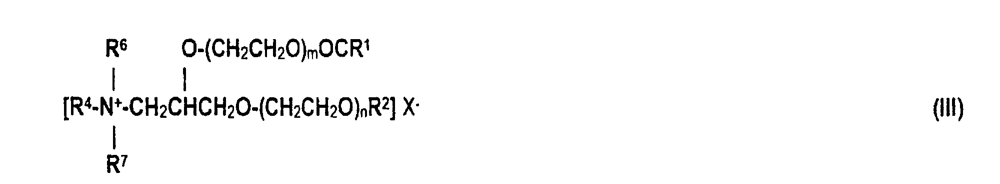 in der R¹CO für einen mit 1 bis 50 Mol Ethylenoxid ethoxylierten Hydroxyacylrest mit 16 bis 22 Kohlenstoffatomen, R² für Wasserstoff oder R¹CO, R⁴, R⁵ und R⁷ unabhängig voneinander für Alkylreste mit 1 bis 4 Kohlenstoffatomen, m und n in Summe für 0 oder Zahlen von 1 bis 12 und X für Halogenid, Alkylsulfat oder Alkylphosphat steht.

4. Esterquats nach den Ansprüchen 1 bis 3, **dadurch gekennzeichnet, daß** sich der Hydroxyacylrest von der Ricinolsäure und/oder der 12-Hydroxystearinsäure ableitet.

5. Verfahren zur Herstellung von ethoxylierten Esterquats, **dadurch gekennzeichnet, daß** man
(a) Hydroxycarbonsäureester mit 16 bis 22 Kohlenstoffatomen im Acylrest mit 1 bis 50 Mol Ethylenoxid ethoxyliert,
(b) die resultierenden ethoxylierten Hydroxycarbonsäureester mit Alkanolaminen oder Alkanolaminethoxylaten umestert und
(c) die resultierenden ethoxylierten Hydroxycarbonsäureesteramine mit Alkylierungsmitteln quaterniert.

6. Verfahren zur Herstellung von ethoxylierten Esterquats, **dadurch gekennzeichnet, daß** man
(a) Hydroxycarbonsäuren mit 16 bis 22 Kohlenstoffatomen mit Alkanolaminen oder Alkanolaminethoxylaten verestert,
(b) die resultierenden Hydroxycarbonsäureesteramine mit 1 bis 50 Mol Ethylenoxid ethoxyliert und
(c) die resultierenden ethoxylierten Hydroxycarbonsäureesteramine mit Alkylierungsmitteln quaterniert.

7. Verfahren zur Herstellung von ethoxylierten Esterquats, **dadurch gekennzeichnet, daß** man
(a) Hydroxycarbonsäuren mit 16 bis 22 Kohlenstoffatomen mit Alkanolaminen oder Alkanolaminethoxylaten verestert,
(b) die resultierenden Hydroxycarbonsäureesteramine mit Alkylierungsmitteln quaterniert und
(c) die resultierenden quaternierten Hydroxycarbonsäureesteramine mit 1 bis 50 Mol Ethylenoxid ethoxyliert.

8. Verfahren nach den Ansprüchen 5 bis 7, **dadurch gekennzeichnet, daß** man als Hydroxycarbonsäuren Ricinolsäure und/oder 12-Hydroxystearinsäure bzw. deren Ester mit Methanol oder Glycerin einsetzt.

9. Verfahren nach mindestens einem der Ansprüche 5 bis 8, **dadurch gekennzeichnet, daß** man Mischungen der Hydroxycarbonsäuren bzw. deren Ester mit weiteren Carboxylverbindungen einsetzt, die ausgewählt sind aus der Gruppe, die gebildet wird von
(a) aliphatischen, linearen oder verzweigten, gesättigten oder ungesättigten Carbonsäuren mit 6 bis 22 Kohlenstoffatomen,
(b) aliphatischen Dicarbonsäuren mit 1 bis 12 Kohlenstoffatomen,
(c) ein- oder mehrwertigen Hydroxycarbonsäuren mit 2 bis 12 Kohlenstoffatomen
sowie deren Mischungen.

10. Verfahren nach Anspruch 9 **dadurch gekennzeichnet, daß** man die Hydroxycarbonsäuren bzw. deren Ester und die Carboxylverbindungen - jeweils bezogen auf die Carboxylgruppen - im molaren Verhältnis 1 : 1 bis 10 : 1 einsetzt.

11. Verfahren nach mindestens einem der Ansprüche 5 bis 10, **dadurch gekennzeichnet, daß** man Anlagerungsprodukte von durchschnittlich 3 bis 30 Mol Ethylenoxid an die genannten Hydroxycarbonsäuren bzw. deren Ester einsetzt.

12. Verfahren nach mindestens einem der Ansprüche 5 bis 11, **dadurch gekennzeichnet, daß** man als Alkanolamine Triethanolamin, Methyldiethanolamin oder deren Mischungen im Molverhältnis 5 : 95 bis 95 : 5 einsetzt.

13. Verfahren nach mindestens einem der Ansprüche 5 bis 12, **dadurch gekennzeichnet, daß** man die Hydroxycarbonsäuren bzw. deren Ester, gegebenenfalls im Gemisch mit den weiteren Carboxylverbindungen und die Alkanolamine im molaren Verhältnis 1 : 1 bis 10 : 1 einsetzt.

14. Verfahren nach mindestens einem der Ansprüche 5 bis 13, **dadurch gekennzeichnet, daß** man als Alkylierungsmittel Methylchlorid, Dimethylsulfat oder Chloressigsäure bzw. deren Salze einsetzt.

15. Verfahren nach mindestens einem der Ansprüche 5 bis 14, **dadurch gekennzeichnet, daß** man die Alkylierung in Gegenwart von niederen Alkoholen als Lösemitteln durchführt.

16. Verfahren nach mindestens einem der Ansprüche 5 bis 14, **dadurch gekennzeichnet, daß** man die Alkylierung in Gegenwart von Fettalkoholen oder Polyolen als Lösemitteln durchführt.

17. Verwendung von Esterquats nach den Ansprüchen 1 bis 3 zur Herstellung von kosmetischen und/oder pharmazeutischen Zubereitungen.

18. Verwendung von Esterquats nach den Ansprüchen 1 bis 3 zur Herstellung von klaren Avivagemitteln.

19. Verwendung von Esterquats nach den Ansprüchen 1 bis 3 zur Herstellung von klaren Flüssigwaschmitteln mit Avivagewirkung.

20. Kosmetische Zubereitungen, enthaltend Esterquats nach den Ansprüchen 1 bis 3 sowie Hilfs- und Zusatzstoffe, ausgewählt aus der Gruppe, die gebildet wird von milden Co-Tensiden, Ölkörpern, Emulgatoren, Überfettungsmitteln, Perlglanzwachsen, Stabilisatoren, Konsistenzgebem, Verdickungsmitteln, Polymeren, Siliconverbindungen, biogenen Wirkstoffen, Antischuppenmitteln, Filmbildnern, Konservierungsmitteln, Hydrotropen, Solubilisatoren, UV-Lichtschutzfiltern, Antioxidantien, Insektenrepellentien, Selbstbräunern, Parfümölen und Farbstoffen.

21. Klare Avivagemittel, enthaltend Esterquats nach den Ansprüchen 1 bis 3 sowie nichtionische Tenside.

22. Klare Flüssigwaschmittel, enthaltend Esterquats nach den Ansprüchen 1 bis 3 sowie nichtionische und/oder anionische Tenside.

## Revendications

1. Esterquats de formule (I) : 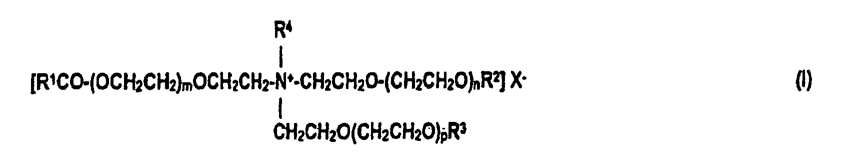 dans laquelle R¹CO, représente un reste hydroxyacyle éthoxylé avec 1 à 50 mol d'oxyde d'éthylène ayant de 16 à 22 atomes de carbone, R², R³ indépendamment l'un de l'autre représente de l'hydrogène ou R¹CO, R⁴ représente un reste alkyle ayant de 1 à 4 atomes de carbone ou un groupe (CH₂CH₂O)qH, m, n et p globalement représentent 0 ou des nombres allant de 1 à 12, q représente des nombres allant de 1 à 12, et X représente un halogénure, un alkylsulfate ou un alkylphosphate.

2. Esterquats de formule (II) : 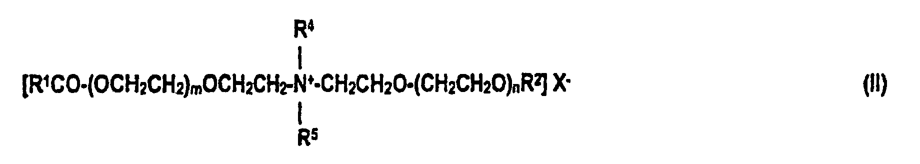 dans laquelle R¹CO représente un reste hydroxyacyle éthoxylé avec 1 à 50 mol d'oxyde d'éthylène ayant de 16 à 22 atomes de carbone, R² représente de l'hydrogène ou R¹CO, R⁴ et R⁵ indépendamment l'un de l'autre représentent des restes alkyle ayant de 1 à 4 atomes de carbone, m et n globalement représentent 0 ou des nombres allant de 1 à 12, et X représente un halogénure, un alkylsulfate ou un alkylphosphate.

3. Esterquats de formule III : 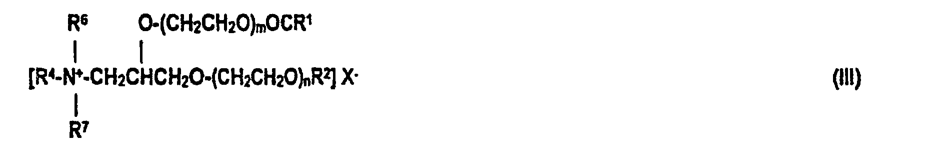 dans laquelle R¹CO représente un reste hydroxyacyle éthoxylé avec de 1 à 50 mol d'oxyde d'éthylène, ayant de 16 à 22 atomes de carbone, R² représente de l'hydrogène ou R¹CO, R⁴, R⁶ et R⁷ indépendamment l'un de l'autre représentent des restes alkyle ayant de 1 à 4 atomes de carbone, m et n globalement représentent 0 ou des nombres allant de 1 à 12 et X représente un halogénure, un alkylsulfate ou un alkylphosphate.

4. Esterquats selon l'une quelconque des revendications 1 à 3,
**caractérisés en ce que**
le reste hydroxyacyle dérive de l'acide ricinoléique et/ou de l'acide 12-hydroxystéarique.

5. Procédé de préparation d'esterquats éthoxylés
**caractérisé en ce qu'**
a) on éthoxyle un ester d'acide hydrocarboxylique ayant de 16 à 22 atomes de carbone dans le reste acyle, avec de 1 à 50 mol d'oxyde d'éthylène,
b) on transestérifie les esters d'acide hydroxycarboxylique éthoxylés résultants avec des alkanolamines ou des éthoxylates d'alkanolamine,
c) on quaternise les aminoesters d'acide hydroxycarboxylique éthoxylés résultants, avec des agents d'alkylation.

6. Procédé de préparation d'esterquats éthoxylés ,
**caractérisé en ce qu'**
a) on estérifie les acides hydroxycarboxyliques ayant de 16 à 22 atomes de carbone, avec des alkanolamines ou des éthoxylates d'alkanolamine,
b) on éthyle l'aminoester d'acide hydroxycarboxylique résultant, avec de 1 à 50 mol d'oxyde d'éthylène,
c) on quaternise l'aminoester d'acide hydroxycarboxylique éthoxylé résultant avec un agent d'alkylation.

7. Procédé de préparation d'esterquats éthoxylés,
**caractérisé en ce qu'**
a) on estérifie les acides hydroxycarboxyliques ayant de 16 à 22 atomes de carbone avec des alkanolamines ou des éthoxylates d'alkanolamine,
b) on quaternise les aminoesters d'acide hydroxycarboxylique résultants avec des agents d'alkylation,
c) on éthoxyle l'aminoester d'acide hydroxycarboxylique quaternisé résultants avec de 1 à 50 moles d'oxyde d'éthylène.

8. Procédé selon l'une quelconque des revendications 5 à 7,
**caractérisé en ce qu'**
on met en oeuvre comme acide hydroxycarboxylique, l'acide ricinoléique et/ou l'acide 12-hydroxystéarique ou leurs esters avec le méthanol ou le glycérol.

9. Procédé selon au moins l'une quelconque des revendications 5 à 8,
**caractérisé en ce qu'**
on met en oeuvre des mélanges d'acide hydroxycarboxylique ou de leur ester avec d'autres composés carboxyliques qui sont choisis dans le groupe formé :
a) des acides carboxyliques aliphatiques linéaires ou ramifiés, saturés ou non saturés, ayant de 6 à 22 atomes de carbone,
b) des acides dicarboxyliques aliphatiques ayant de 1 à 12 atomes de carbone,
c) des acides hydroxycarboxyliques uni- ou plurivalents ayant de 2 à 12 atomes de carbone, ainsi que de leurs mélanges.

10. Procédé selon la revendication 9,
**caractérisé en ce qu'**
on met en oeuvre les acides hydroxycarboxyliques ou leurs esters et les composés carboxyliques dans le rapport molaire de 1:1 à 10 :1 à chaque fois rapporté aux groupes carboxyliques.

11. Procédé selon au moins l'une quelconque des revendications 5 à 10,
**caractérisé en ce qu'**
on met en oeuvre des produits d'addition de en moyenne 3 à 30 mol d'oxyde d'éthylène sur les acides hydroxycarboxyliques ou leurs esters cités.

12. Procédé selon au moins l'une quelconque des revendications 5 à 11,
**caractérisé en ce qu'**
on met en oeuvre comme alkanolamine de la triéthanolamine, de la méthyldiéthanolamine, ou leurs mélanges dans un rapport molaire de 5 :95 à 95 :5.

13. Procédé selon au moins l'une quelconque des revendications 5 à 12,
**caractérisé en ce qu'**
on met en oeuvre les acides hydroxycarboxyliques ou leurs esters, le cas échéant en mélange avec les autres composés carboxyliques et les alkanolamines dans un rapport molaire de 1 :1 à 10 :1.

14. Procédé selon au moins l'une quelconque des revendications 5 à 13,
**caractérisé en ce qu'**
on met en oeuvre comme agent d'alkylation, du chlorure de méthyle, de sulfate de diméthyle ou de l'acide chloracétique ou ses sels.

15. Procédé selon au moins l'une quelconque des revendications 5 à 14,
**caractérisé en ce qu'**
on effectue l'alkylation en présence d'alcools inférieurs comme solvants.

16. Procédé selon au moins l'une quelconque des revendications 5 à 14,
**caractérisé en ce qu'**
on effectue l'alkylation en présence d'alcools gras ou de polyols comme solvants.

17. Utilisation des esterquats selon l'une quelconque des revendications 1 à 3, pour la production de préparations cosmétiques et ou pharmaceutiques .

18. Utilisation des esterquats selon l'une quelconque des revendications 1 à 3, pour la production d'agents d'avivage limpides.

19. Utilisation des esterquats selon l'une quelconque des revendications 1 à 3, pour la production de produits de lavage liquides, limpides ayant une action avivante.

20. Préparations cosmétiques contenant des esterquats selon l'une quelconque des revendications 1 à 3 ainsi que adjuvants et des additions choisis dans le groupe formé de co-tensioactifs doux, de composés huileux, d'agents émulsionnants, d'agents de surgraissage de cires à éclat nacré, d'agents stabilisants, de produits qui donnent de la consistance, d'agents épaississants, de polymères, de composés de silicone, de principes actifs biogènes, d'agents antipelliculaires, d'agents filmogènes, d'agents conservateurs, de substances hydrotropes, d'agents de solubilisation, de filtres contre la lumière UV, d'agents antioxydants, de répulsifs pour les insectes, de produits autobronzants, d'essence de parfum et de colorants.

21. Procédé d'avivage limpides contenant des esterquats selon l'une quelconque des revendications 1 à 3, ainsi que des agents tensioactifs non ioniques.

22. Produits de lavage liquides, limpides contenant des esterquats selon l'une quelconque des revendications 1 à 3 ainsi que des agents tensioactifs non ioniques et/ou anioniques.

## Claims

1. Esterquats corresponding to formula **(I):** 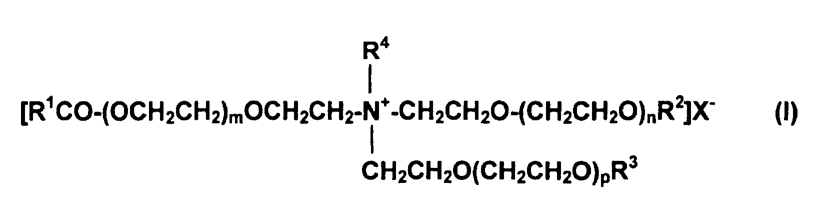 in which R¹CO is a hydroxyacyl group containing 16 to 22 carbon atoms ethoxylated with 1 to 50 mol ethylene oxide, R² and R³ independently of one another represent hydrogen or have the same meaning as R¹CO, R⁴ is an alkyl group containing 1 to 4 carbon atoms or a (CH₂CH₂O)_{q}H group, m, n and p together stand for 0 or numbers of 1 to 12, q is a number of 1 to 12 and X is halide, alkyl sulfate or alkyl phosphate.

2. Esterquats corresponding to formula **(II):** 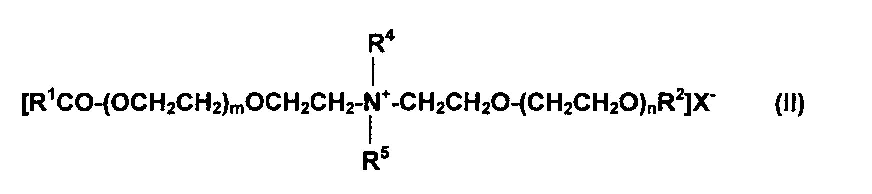 in which R¹CO is a hydroxyacyl group containing 16 to 22 carbon atoms ethoxylated with 1 to 50 mol ethylene oxide, R² is hydrogen or has the same meaning as R¹CO, R⁴ and R⁵ independently of one another are alkyl groups containing 1 to 4 carbon atoms, m and n together stand for 0 or numbers of 1 to 12 and X stands for halide, alkyl sulfate or alkyl phosphate.

3. Esterquats corresponding to formula **(III):** 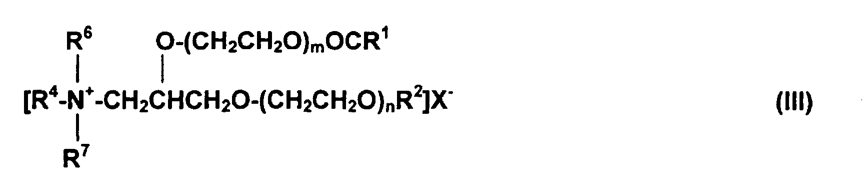 in which R¹CO is a hydroxyacyl group containing 16 to 22 carbon atoms ethoxylated with 1 to 50 mol ethylene oxide, R² is hydrogen or has the same meaning as R¹CO, R⁴, R⁶ and R⁷ independently of one another are alkyl groups containing 1 to 4 carbon atoms, m and n together stand for 0 or numbers of 1 to 12 and X stands for halide, alkyl sulfate or alkyl phosphate.

4. Esterquats as claimed in claims 1 to 3, **characterized in that** the hydroxyacyl group is derived from ricinoleic acid and/or 12-hydroxystearic acid.

5. A process for the production of ethoxylated esterquats, **characterized in that**
(a) hydroxycarboxylic acid esters containing 16 to 22 carbon atoms are ethoxylated with 1 to 50 mol of ethylene oxide,
(b) the resulting ethoxylated hydroxycarboxylic acid esters are transesterified with alkanolamines or alkanolamine ethoxylates and
(c) the resulting ethoxylated hydroxycarboxylic acid ester amines are quaternized with alkylating agents.

6. A process for the production of ethoxylated esterquats, **characterized in that**
(a) hydroxycarboxylic acids containing 16 to 22 carbon atoms are esterified with alkanolamines or alkanolamine ethoxylates,
(b) the resulting hydrocarboxylic acid ester amines are ethoxylated with 1 to 50 mol of ethylene oxide and
(c) the resulting ethoxylated hydroxycarboxylic acid ester amines are quaternized with alkylating agents.

7. A process for the production of ethoxylated esterquats, **characterized in that**
(a) hydroxycarboxylic acids containing 16 to 22 carbon atoms are esterified with alkanolamines or alkanolamine ethoxylates,
(b) the resulting hydroxycarboxylic acid ester amines are quaternized with alkylating agents and
(c) the resulting quaternized hydroxycarboxylic acid ester amines are ethoxylated with 1 to 50 mol of ethylene oxide.

8. A process as claimed in claims 5 to 7, **characterized in that** ricinoleic acid and/or 12-hydroxystearic acid or esters thereof with methanol or glycerol are used as the hydroxycarboxylic acids.

9. A process as claimed in at least one of claims 5 to 8, **characterized in that** mixtures of the hydroxycarboxylic acids or their esters with other carboxyl compounds selected from the group consisting of
(a) aliphatic, linear or branched, saturated or unsaturated carboxylic acids containing 6 to 22 carbon atoms,
(b) aliphatic dicarboxylic acids containing 1 to 12 carbon atoms,
(c) mono- or polybasic hydroxycarboxylic acids containing 2 to 12 carbon atoms
and mixtures thereof are used.

10. A process as claimed in claim 9, **characterized in that** the hydroxycarboxylic acids or their esters and the carboxyl compounds are used in a molar ratio of 1:1 to 10:1, based on the carboxyl groups.

11. A process as claimed in at least one of claims 5 to 10, **characterized in that** products of the addition of, on average, 3 to 30 mol of ethylene oxide onto the hydroxycarboxylic acids mentioned or their esters are used.

12. A process as claimed in at least one of claims 5 to 11, **characterized in that** triethanolamine, methyl diethanolamine or mixtures thereof in a molar ratio of 5:95 to 95:5 are used as the alkanolamines.

13. A process as claimed in at least one of claims 5 to 12, **characterized in that** the hydroxycarboxylic acids or their esters, optionally in admixture with the other carboxyl compounds, and the alkanolamines are used in a molar ratio of 1:1 to 10:1.

14. A process as claimed in at least one of claims 5 to 13, **characterized in that** methyl chloride, dimethyl sulfate or chloroacetic acid or salts thereof are used as alkylating agents.

15. A process as claimed in at least one of claims 5 to 14, **characterized in that** the alkylation is carried out in the presence of lower alcohols as solvents.

16. A process as claimed in at least one of claims 5 to 14, **characterized in that** the alkylation is carried out in the presence of fatty alcohols or polyols as solvents.

17. The use of the esterquats claimed in claims 1 to 3 for the production of cosmetic and/or pharmaceutical preparations.

18. The use of the esterquats claimed in claims 1 to 3 for the production of clear conditioners.

19. The use of the esterquats claimed in claims 1 to 3 for the production of clear liquid detergents with a conditioning effect.

20. Cosmetic preparations containing the esterquats claimed in claims 1 to 3 and auxiliaries and additives selected from the group consisting of mild co-surfactants, oil components, emulsifiers, superfatting agents, pearlizing waxes, stabilizers, consistency factors, thickeners, polymers, silicone compounds, biogenic agents, antidandruff agents, film formers, preservatives, hydrotropes, solubilizers, UV protection factors, antioxidants, insect repellents, self-tanning agents, perfume oils and dyes.

21. Clear conditioners containing the esterquats claimed in claims 1 to 3 and nonionic surfactants.

22. Clear liquid detergents containing the esterquats claimed in claims 1 to 3 and nonionic and/or anionic surfactants.
